(19) **Europäisches Patentamt**

**European Patent Office**

**Office européen des brevets**

(11) Publication number : **0 292 495 B1**

(12) # EUROPEAN PATENT SPECIFICATION

(45) Date of publication of patent specification :
18.09.91 Bulletin 91/38

(51) Int. Cl.⁵ : **A61K 31/17, A61K 31/045,**
**A61K 31/765, A61K 7/04,**
**A61K 7/48, A61K 47/00**

(21) Application number : **87901161.7**

(22) Date of filing : **04.02.87**

(86) International application number :
**PCT/SE87/00053**

(87) International publication number :
**WO 87/04617 13.08.87 Gazette 87/18**

(54) PHARMACEUTICAL COMPOSITIONS CONTAINING PROPYLENE GLYCOL AND/OR POLYETHYLENE GLYCOL AND UREA AS ACTIVE MAIN COMPONENTS.

(30) Priority : **04.02.86 SE 8600501**

(43) Date of publication of application :
**30.11.88 Bulletin 88/48**

(45) Publication of the grant of the patent :
**18.09.91 Bulletin 91/38**

(84) Designated Contracting States :
**AT BE CH DE FR GB IT LI LU NL SE**

(56) References cited :
EP-A- 0 138 029
DE-B- 1 911 144
DE-B- 2 608 221
GB-A- 2 086 223
GB-A- 2 116 425
US-A- 3 395 236
Patent Abstracts of Japan, abstract of JP
Kokai no. 52-87235, published 20.07.1977

(56) References cited :
DERMATOLOGICA, vol. 169, suppl. 1, issued 1984 (Basel, CH), S. Nolting "Non-traumatic removal of the nail and simultaneous treatment of onychomycosis", pages 117-120
ARCHIVES OF DERMATOLOGY, vol. 113, issued March 1977, Chigago, US, F.K. Bagatell, "Topical therapy for onychomycosis", see page 378
Kärnan, Volume 9, no. 1, issued 1985 (AB Leo, Helsingborg, SE), J. Faergemann "Pityriasis versicolor. Orsak, diagnos och behandling", see pages 13-17

(73) Proprietor : **MOBERG, Sven**
**Box 2057**
**S-433 02 Partille (SE)**

(72) Inventor : **MOBERG, Sven**
**Box 2057**
**S-433 02 Partille (SE)**

(74) Representative : **Bergvall, Stina-Lena et al**
**Dr. Ludwig Brann Patentbyra AB P.O. Box 17192**
**S-104 62 Stockholm (SE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compositions for the treatment of hyperkeratotic skin diseases, seborrheic eczema (mainly of the scalp), mycosis of the skin, nails and thickened and chapped skin (rhagades).

The composition consists of a mixture of propylene glycol (optionally polyethylene glycol) and urea as pharmacologically active main components, optionally in combination with lactic acid for the treatment of nails and skin and with the further addition of glycerol and gel-forming agents for the treatment of skin and alcohol for the treatment of the scalp.

Propylene glycol is used in concentration between 40 and 80 percent by weight, urea in concentrations between 5 and 20 percent by weight and lactic acid in amounts up to 20 percent by weight, glycerol up to 10 percent by weight, gel forming agents up to 5 percent by weight and alcohol up to 55 percent by weight.

It is previously known that propylene glycol is a favourable solvent and penetration promoting agent for other pharmaclologically active substances. It is also a very common solvent and is used in several drugs, above all in topical preparations. Since propylene glycol is a common solvent it can also be used to dissolve urea (US-A 3395236 and GB-A 2116425, DE-B 1911144 and DE-A 2847975 and FI-B 56486). For this purpose propylene glycol is preferably used in concentrations below 10%, very occasionally up to 40%, when e.g. propylene glycol is included in combination with fatty acids and about 0.02% urea (US-A 3395236).

Propylene glycol has also been shown to be effective against one type of yeast fungi, Pityrosporum orbiculare, giving a specific skin disease Pityriasis versicolor. An aqueous solution of 50% propylene glycol has been shown to give some improvement.

The present invention, using propylene glycol in high concentrations (over 40%), in combination with urea and optionally lactic acid, exploits the unique more potent inherent effects of the combinations against mycosis of the nails and dermatomycoses as well as hyperkeratotic skin diseases and seborrheic eczema and also thickened and chapped skin.

### Propylene glycol

Propylene glycol is a water-soluble, odourless, colourless, atoxic solvent, which is used as a vehicle in several topical and oral preparations. The agent is also given in the form of injections. Characterizing features for propylene glycol are i.a. its slightly keratolytic stratum corneum disintegrating and water binding properties. The agent has also an antimycotic and antibacterial effect (5, 6, 7, 8, 9, 10).

In some cases a local irritant effect has been reported as a side effect (6) with an increased concentration of the solvent. Allergic reactions are also occasionally reported (10).

The agent is also used experimentally on larger skin areas in connection with yeast fungi infections (pityriasis versicolor) with favourable results (7). It has been shown to potentiate the effect of other simultaneously added drugs, (5, 6), in connection with dermatomycosis but separately it has a weaker effect, (6). This also applies when treating nails (11).

The concentration of propylene glycol is, however, low when used in topical preparations. Propylene glyclol is then included in order to dissolve other substances. Its penetration promoting properties are utilised in combination with other potent substances, e.g. steroids. Thus, several steroid preparations for local application contain propylene glycol.

The potent keratolytic and fungicidal properties which, characterize the invention are obtained with propylene glycol in a high concentration of about 70% :

a) in combination with urea, lactic acid and glycerol as well as gel-forming agents for the treatment of hyperkeratotic skin diseases, and

b) in combination with urea and lactic acid for the treatment of mycosis of the nails.

Thus, propylene glycol is included in the claimed preparation as an active substance in combination with other active substances (e.g.) urea, lactic acid) and not only as a solvent thereof.

### Urea

Urea is a reliable, atoxic, water soluble substance for the treatment of the skin and scalp. Urea is i.a. included in Calmuril and HTH®. The Calmuril solution is registered for the indication seborrheic eczema. Urea has antibacterial, antimycotic, protein-dissolving, keratolytic, water-binding and antipruritic properties, (11, 13, 14) and also has penetration promoting properties and a low pH-value (15, 2). Urea in concentrations of 20 to 40% has a nail-dissolving effect under occlusion, (13, 14). According to the method as hitherto used, the nail is covered with a paste of urea under occulsion for one week. (13). The method requires skilled staff. Side effect reported from the use of urea are i.a. a temporary stinging effect.

Urea may also be included in combination with substances with an antimycotic effect. An antimycotic agent (bifonazol) Mycosporan® is currently being tested in combination with urea for the treatment of mycosis of the nails (15).

Acidum lacticum (lactic acid) Lactic acid has i.a. an acidifying effect.

Example

Preparation

| I. | Urea | 20 % |
| | Lactic acid | 10 % |
| | Propylene glycol | 70 % |

| II. | Urea | 20 % |
| | Propylene glycol | 80 % |

Preparation

| III. | Urea | 20 % |
| | Propylene glycol | 40 % |
| | Alcohol (70 %) | 40 % |

| IV. | Urea | 20 % |
| | Propylene glycol | 40 % |
| | Lactic acid | 10 % |
| | Alcohol (70 %) | 30 % |

Preparation *)

| V. | Urea | 15 % |
| | Lactic acid | 10 % |
| | Glycerol | 5 % |
| | Propylene glycol | 70 % |

Control treatment:

| Lactic acid | 10 % |
| Propylene glycol | 90 % |

Preparations I and II were used for the treatment of mycosis of the nails, preparations III and IV for the treatment of seborrheic eczema on the scalp and preparation V for treatment of the skin.

The proportional ratios between the substances stated above are not absolute but can be varied within the limits stated in the claims and the preparations can be used without limitation for all of the indications stated even if the test is performed on specific indications.

A composition containing urea and propylene glycol for the treatment of nail fungus.

Mycosis of the nails is a common complaint which gives stained, sometimes thickened and fragmented nails. Nails can be infected by dermatophytes, yeast, fungi, or moulds, but bacteria may also be contributory (1).

Preparations recorded for the treatment of mycosis of the nails by local application are imidazole deriva-

*) To the preparation for skin treatment a gel-forming agent was added in an amount of 0.5-5%, preferably 0.5-3% by weight, in order to obtain a better consistency. E.g. klucel, Carbomer® 940 or Macrogol® 6000 or other gel forming agents can be used.

tives, e.g. Canesten®, Pevaryl®, Daktar® and previously also a combination preparation Onycho-Phytex, which, however, has a comparatively poor healing effect.

The rate of healing as obtained with a griseofulvin (Fulcin®) is low despite a long duration of treatment (2). Further, the agent is only effective against dermatophytes and lacks effect against yeasts, moulds and bacteria.

Nor is the currently registered drug ketaconazole (Fungoral[R]) suitable for the treatment of mycosis of the nails separately, due to the side effects of the preparation.

It is a well-known fact that mycosis of the nails and especially toe nails is more difficult to treat than many other forms of mycotic infections. Inferior results are, as mentioned, obtained by topical as well as peroral treatment. The period of treatment is calculated to be between 6 and 12 months. Topical preparations for mycosis of the nails is furthemore poorly documented. Doctors currently advise patients to refrain from treatment of toe-nails due to the poor results of treatment.

Review of clinical investigations

The investigation was performed double blind after randomisation by comparison of two solutions, one containing a combination of 20% urea, 10% lactic acid and 70% propylene glycol (solution A) (example I) and the other containing propylene glycol 90% in combination with lactic acid 10% (solution B) (control).

Urea is readily soluble at a concentration of 10-20% in propylene glycol. By the addition of lactic acid, an increased acidity is obtained, which is favourable since a low pH-value inhibits the growth of the fungi in the nail.

Liquid preparations were applied in the form of drops and penetrated in that form into the nail substance. The treatment was cosmetically well accepted.

The poor effect of many nail treatment agents is probably due to the fact that the substances do not effectively penetrate the nail infected by fungi. An increased penetration of the nail substance is obtained with the new composition of urea, propylene glycol and lactic acid. The antimycotic effect might be enhanced by the addition of other pharmacologically active substances.

Twenty-one (21) patients with chronic mycosis of the nails participated in a clinical test. 68 nails infected by fungi were treated (58 toe nails and 10 finger nails). Culturing before the treatment showed in each case growth of Trichophyton rubrum from the nails. All of the nails were heavily infected by fungi with thickened nails, discolouration, onycholysis (loosening of the nail plate) and partial fragmentation of the nail plate.

The nails were divided into two groups :

Group A received solution A (urea and propylene glycol as the main substances)

Group B received solution B (propylene glycol as the main substance).

The duration of the complaint (average value) was 3 years 10 months (1-10 years).

Treatments were previously performed locally with imidazolesolutions (Pevaryl or Canesten) on average for 6 months (1-24) months. Only 3 of 21 patients reported an improvement. 4 of 21 stated that the nails had become softer, 1 of 21 reported a deterioration and 13 of 21 considered the previous treatment to be without effect. The patients were therefor motivated for new treatment.

The nail area attacked was on average 88% (group A) and 81% (group B) respectively, before the treatment with the claimed drug. The solutions were applied dropwise to the nails (2-5 drops per nail) once daily. During the first month the nail plate was also covered with non allergic plaster after each application of the nail agent.

Results

The results were documented by photo before and during the treatment (after 14 days and then once a month) and according to record.

An obvious improvement was recorded after only 14 days. The nail plate was also softer in group A (urea-propylene glycol) than in group B (propylene glycol). Parts of the nails attacked were in group A almost dissolved. The result was recorded as percent of the nail area cured after one and two months respectively (mean values) :

|  | 1 month | 2 months |
|---|---|---|
| Solution A (urea-propylene glycol): | 48 % | 59% |
| Solution B (propylene glycol): | 22 % | 39 % |

≥ 80% of clean nail area was recorded after treatment for two months for 53% (16 of 30) of the nails in group

4

A and for 26% (10 of 38) of the nails in group B.

$\geq$ 90% clean nail area was recorded after the same treatment period for 30% (9 of 30) of the nails in group A and for 5% (2 of 38) of the nails in group B. One nail (group A) was completely healed during this period.

Thus, urea-propylene glycol had a significantly better effect than propylene glycol only. Furthermore, the softening effect was more pronounced with urea-propylene glycol.

A follow-up examination after terminated treatment will be performed later on. Since the treatment period for nails is calculated to be at least 6 months, continued treatment can be expected to give better treatment results. Complete healing of the nail plate cannot always be expected, due to matrix damage caused by the mycotic infection (14).

Side effects

Two patients reported a slight stinging effect and tenderness around the nail during one day. Three patients reported slight redness of the skin around the nail during one to three days. One patient also observed desquamation of the skin around the nail.

Conclusions

By combining 20% of urea with a high concentration of propylene glycol (70%) and lactic acid (10%), a new and previously untested composition for the treatment of onycho mycosis which has yielded unexpectedly good results is obtained. It has been shown that the combination is more effective and has a better softening and more potent keratolytical (stratum corneum disintegrating) effect than propylene glycol alone. By combining propylene glycol and urea a penetrating effect is also obtained. This effect can be increased by occlusion with non allergic plaster around the nail. The combination of propylene glycol and urea is believed to be a solvent which promotes the penetration of other substances having an antimycotic action. It should be that it is commonly known among men skilled in the art, and supported by investigations, that preparations solely containing urea have a very poor effect on mycosis of the nails.

Nail lesions from other causes

Chronic onychia with impressions in the nail plate can be improved and debris under psoriatic nails can be removed with a solution of urea in propylene glycol in the same concentration (cf. pictures).

Compositions containing urea and propylene glycol with the addition of glycerol and acidum lacticum (lactic acid) for the treatment of skin diseases characterized by hyperkeratosis and scalling

A potent keratolytic and softening action superior to that of other commercial preparations or extempore preparations containing e.g. urea or salicylic acid is achieved by combination of urea with propylene glycol at high concentration. The softening properties are enhanced by the addition of glycerol and lactic acid. The preparation also prevents the reddening of the skin (e.g. by psoriasis) and has a curative effect on inflammatory (erythematous) forms of eczema, which seems paradoxical in view of the often irritating effect of propylene glycol at high concentrations. The substances as included are all known and reliable. The novelty lies in 1) the composition of the preparation which gives

a) an unexpectedly good effect of the treatment,

b) pharmaceutical properties where certain particulars are concerned with the solubility of the substances and so on. Also 2) the indications of the claimed composition are new (cf. the report on the results. The combination of substances included should, theoretically have an effect on hyperkeratotic skin diseases and scalling skin diseases by the properties of the substances known per se. However, the rapidly occuring good effect was quite unexpected and might be explained by a synergistic effect between the included substances, the said substances being well balanced in relation to each other.

Urea in a concentration of 10-20% has shown good solubility in propylene glycol. An enhanced acidifying effect, desired in topical skin preparations, is obtained by the addition of lactic acid.

Advantages obtained with the claimed preparation in the treatment of hyperkeratotic skin diseases

1. The preparation is cosmetically attractive, since it does not smear or soil, is odourless, penetrates the skin easily and has a low pH-value, which is favourable.

2. The substances as included are kind to the skin and rarely give side effects (cf. under Side effects).

3. The preparation is an alternative for patients who desire cortisone-free treatment since the preparation lacks the side effects of cortisone, e.g. skin atrophy (attentuation) (valid mainly for group III-IV steroids).

4. The preparation is easy to manufacture, and the raw materials are relatively cheap (cf. under Manufacturing process).

5. The preparation is stable without the addition of preservatives (cf. under the title Stability test. The said test relates to an analysis where the concentration of urea was unchanged after a shelf-life of three months).

6. The preparation has antibacterial and antimycotical properties.

7. Other substances can also be combined with the composition, e.g. hydrocortisone, or fluorinated corticosteroides in an amount of up to 2%, which gives several advantages e.g. by increasing the penetration properties of the steroid. antimycotical substances such as e.g. imidazole derivatives in amounts up to 2%, collodion 0-10%, salicylic acid 0-10%, vitamin A 0-1%, vitamin A-acid 0-1% (by weight) and derivatives thereof.

8. One advantage of the preparations according to the claims is that, in addition to being pharmaceutically active in the amounts defined in the claims, they can also act as solvents for other pharmaceutically active substances such as e.g. the substances stated above.

A report on the results of treatment of chronic hyperkeratotic skin diseases

Examples of diseases that can be treated are :
psoriasis
pustulosis palmoplantaris
tylotic eczema
hyperkeratotic eczema
neurodermatitis
ichthyosis
keratoderma
mycotic infections
clavi (corns)

A total of 40 patients are of present being treated.

Symmetrically located similar skin lesions were chosen for the treatment. One side was treated with the claimed composition consisting of urea/propylene glycol, lactic acid, glycerol and a gel-forming agent (example V), and the other side with a potent corticosteroid (group III) and a known emollient preparation containing salicylic acid 2-5% or urea 10% (control side).

Results

The preliminary results, which were documented by photos, were so favourable that conclusions with respect to the effect of the treatment could already be drawn at an early stage. The effect of the claimed composition was unexpected and in some cases almost dramatic. A potent desquamation of the skin gave in all cases, often after only 14 days, a remarkable improvement and in certain cases complete cure after a treatment period of one month. The effect was more rapid and better than that given by the control treatment. The treatment gave best results in hyperkeratotic eczema, chronic mycotic infections and psoriasis. These complaints were reported separately. The preparation also has an excellent effect on dry skin and chaps.

Side effects : a slight stinging effect was noted in individual cases.

A. Hyperkeratotic eczema

These are characterized by thickened, scaling skin, often with chaps (rhagades). Eczema on the palms and the soles was treated.

Results

Very favourable results, with a rapid, unilateral peeling effect and a remarkable improvement in the eczema, were noted with the claimed preparation. Remarkably, painful, deep rhagades (chaps) were also cured and the pain disappeared. The healing period was on average about one month. The control side showed inferior results.

B. Chronic mycotic infections

Mycotic infections were caused by a dermatophyte, commonly Trichophyton rubrum. The symptoms are often dry chapping of the palms and the soles. The infections are, as a rule, chronic, and therefore difficult to treat and frequently recurrent.

Results

Remarkably good results, with rapid desquamation and apparantly healed skin after about one months treatment are achieved with the claimed preparation. The combination of rapid desquamation of infected skin cells, the antimycotic effect of the preparation and its low pH-value probably contribute to the effect. These three properties are achieved by the well-balanced, exact composition of the preparation. These properties together make the preparation new in relation to other reliable antimycotic agents. No stinging effect was noted during treatment of the palms and soles but this, might be a problem if the agent is used on discharging or reddened skin areas or in the groin. Other methods of treatment are currently recommended for this purpose. Thus, the main indication for treatment of dermatomycosis is chronic infection of the palms and soles or dry, scalling mycotic attacks on glabrous skin.

Pityriasis versicolor

This condition is caused by a yeast fungus, Pityrosporon orbiculare, which can also be treated with the preparation according to the invention. The treatment of the complaint with 50% propylene glycol in aqueous solution has been tested previously and is thus previously known (7).

Conclusion

Novelty : the composition of the claimed preparation in the indication dry eczema and mycotic infections (dermatophytosis). The invention is not obvious in view of the unexpectedly favourable effect of the product.

C. Psoriasis

Psoriasis is a skin disease characterized by reddened, flaking skin lesions. The disease has, as a rule, a chronic course. Topical agents for the treatment of psoriasis are often smeary and discolouring, e.g. dithranol or tar. Steroid preparations are cosmetically attractive but can give the side effects of cortisone e.g. in the form of skin attenuation. Emollient preparations containing for example salicylic acid and urea in a cream or ointment base have a certain peeling effect but are not separately sufficiently active. A cosmetically acceptable topical agent having a more potent effect and lacking the side effects of cortisone for the treatment of psoriasis is not currently available.

Symmetrically situated, well defined chronic psoriasis lesions were chosen for the treatment in order to evaluate more critically the effect of the claimed preparation. One side was treated twice a day with the claimed preparation and the other side with a common emollient treatment and a potent steroid ointment (group III), the latter twice a day.

Result

The result of the treatment was totally unexpected. A very rapid and pronounced improvement was noted after only fourteen days of treatment with the claimed preparation. A treatment period of one month resulted in many cases in complete healing of the area treated. The effect was comparable to or better than, that obtained on the control side, which was treated with a potent (group III) steroid. In addition to a keratolytic effect, it was also noted that the erythema receded. The anti-psoriatic effect is difficult, to explain in the light of present knowledge about the separate components as included. These components might have a synergistic effect and might also simultaneously have an anti-inflammatory or antimitotic effect.

Side effects

A slight stinging effect was noted in some cases (common for urea). The patients considered the cosmetic properties of the preparation to be very favourable (cf. page 12). The rapid desquamative properties of the preparation are also very important, e.g. in combination with treatment with light.

## Conclusions

The said composition is new and the indication also differs from what is known by EP-A-0138029. The results of the treatment were very favourable, with an unexpected, rapid keratolytic and anti-psoriatic effect comparable with that of potent corticosteroids and more effective than that obtained with common emollient treatment of the type urea in a cream base.

The preparation according to the invention provides a very valuable addition to the therapeuticarsenal for psoriasis patients since it is cosmetically attractive and efficacious.

## Further indications :

seborrheic eczema
verrucae

## Seborrheic eczema

Seborrheic eczema is a common complaint with itching, erythema and scaling on the scalp. The eczema is often also located on the face, in the armpits and in the groin. The hitherto most common methods of treatment have been different steroid (cortisone) preparations which provide symptomatic alleviation by having a temporary antipruitic and anti-inflammatory effect.

The etiology of seborrheic eczema is commonly discussed and a relatively new hypothesis is that a yeast fungus (Pityrosporon ovale/orbiculare), may be one of the causes. (3, 4).

A new method of treating seborrheic eczema would be the use of a drug which inhibits the yeast fungus growth and thereby not only gives symptomatic alleviation but also eliminates the causes. Propylene glycol has such an inhibitory effect.

There is, therefore, a demand for alternative preparations intended for the treatment of seborrheic eczema.

An alternative preparation for the treatment of seborrheic eczema is now offered by the present invention in which propylene glycol and with urea are used as active substances, optionally in combination with other active substances.

Some test cases of seborrheic eczema of the scalp treated with compositions III and IV are reported in the Examples.

The treatment is effective but the symptoms recur about one to two weeks after the end of the treatment. Two courses of treatment per week are often enough for maintenance treatment. The solution is in these cases washed out after about five to twelve hours. The preparation for the hair can give a somewhat greasy feeling to the hair and scalp but this can be an advantage with dry scalps. The preparation according to the invention is an alternative for the patients who wish to avoid steroid treatment (cortisone preparations) for their eczema. No side effects of the preparation have been observed.

## Verrucae

A certain effect was also obtained when verrucae were treated with the solution used for mycosis of the nails, which solution contained urea, propylene glycol and lactic acid. The difficulty in making the preparation remain on the verrucae might be eliminated by the use of for example collodion.

The claimed preparation, has due to its composition, shown unique properties though its effect on a great number of skin diseases having different etiology. This is very uncommon in a medical context. It has further been shown that the combination (urea-propylene glycol) has a more potent keratolytic (stratum corneum disintegrating) effect than propylene glycol alone. The combination is also, effective for nail mycosis.

In addition to its keratolytic properties, the skin preparation also has an anti-psoriatic effect and in some cases also an anti-erythematous effect. This is surprising and cannot be explained by the mechanisms of action of each of the separate substances. The combined treatment has also shown a better effect than preparations containing urea only and in many cases also potent corticosteroids (group III).

## Manufacturing example 1 :

| | |
|---|---|
| Urea | 20% |
| Lactic acid | 10% |
| Propylene glycol ad. | 100% |

Preparation directions :

The urea is dispersed as finely as possible and mixed with a minor amount of propylene glycol. The remainder of the propylene glycol is then added and all of it stirred together until the urea is dissolved (time consuming). Finally, lactic acid is added.

Manufacturing example 2 :

| Gel-forming agent | 0.5-3% |
|---|---|
| Glycerol 85% | 5% |
| Lactic acid | 10% |
| Urea | 15% |
| Propylene glycol ad. | 100% |

Preparation directions :

The urea is dispersed as finely as possible and mixed with a minor amount of propylene glycol. The remainder of the propylene glycol is then added and all of it stirred together until the urea has dissolved (time consuming). The glycerol and the lactic acid are added.

A gel-forming agent is mixed into a small amount of the above-mentioned solution to a homogeneous mixture, after which the remainder of the solvent is added and everything is stirred until a homogeneous gel is formed (time consuming).

Stability Test

A stability test was performed by the Central Laboratory of Apoteksbolaget, Solna, on a preparation with the composition :

20% urea
70% propylene glycol
10% lactic acid

Results of analysis after three months of three samples of the preparation in which the amounts of urea, which is the chemically unstable component, should by 18-22%.

```
Vial 1.    20.5 %
 "   2.    19.9 %
 "   3.    19.9 %
```

1. Zaias N. Onychomycosis. Arch Derm 1972 ; 105 : 263-74.
2. Ishii M, Hamada T, Asai Y. Treatment of onychomycosis by ODT therapy with 20% urea ointment and 2% tolnaftate ointment. Dermatologica 1983 ; 167 : 273-9.
3. Priestley G C. Sevin J A. The microbiology of dandruff. Br J Dermatol 1976 ; 94 : 469-71.
4. Shuster S. The aetiology of dandruff and the mode of action of therapeutic agents. Br J Dermatol 1984; III : 235-42.
5. Nielsen P G. The importance of the vehicle in the treatment of dermatophytosis in hereditary palmo-plantar keratoderma. Mykosen 1984 ; 27 (5) : 227-30.
6. Gamborg Nielsen P. Dermatophyte infections in hereditary palmo-planter keratoderma. Dermatologica 1984 ; 168 : 238-41.
7. Faergemann J. Fredriksson T. Propylene glycol in the treatment of tinea versicolor. Acta Derm Venereol 1979 ; 60 : 92-3.
8. Faergemann J. Bernander S. The activity in vitro of five different antimycotics against pityrosporum orbiculare. Acta Derm Venereol 1979 ; 59 : 521-4.
9. Leyden J J. Stewart R, Kligman A M. Updated in vivo methods of evaluating topical antimicrobial agents on human skin. J. Invest Dermatol 1979 ; 72 : 165-70.
10. Martindale, The extra pharmacopoeia, Ed. 28, 1982.

11. Bagatell F K. Topical therapy for onychomycosis. Arch Derm 1977 ; 113 : 378.

12. Ashton H, Frenk E, Stevenson C J. Urea as a topical agent. Br J Dermatol 1970 ; 84 : 194-6.

13. Farber E M, South D A. Urea ointment in the nonsurgical avulsion of nail dystrophies. Cutis 1978 ; 22: 689-92.

14. South D A, Farber E M. Urea ointment in the nonsurgical avulsion of nail dystrophies — a reappraisal. Cutis 1980 : 25 : 609-12.

15. Nolting S. Non-traumatic removal of the nail and simultaneous treatment of onychomycosis. Dermatologica 1984 ; 169 (suppl I) : 117-20.

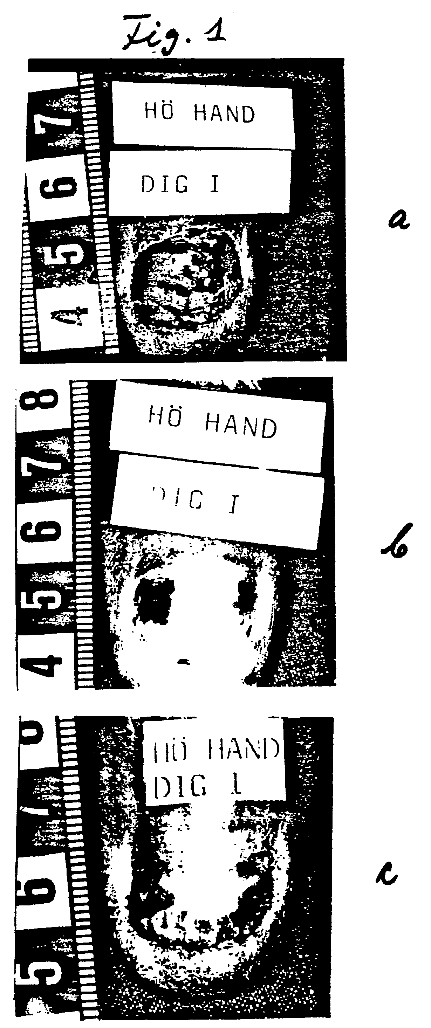

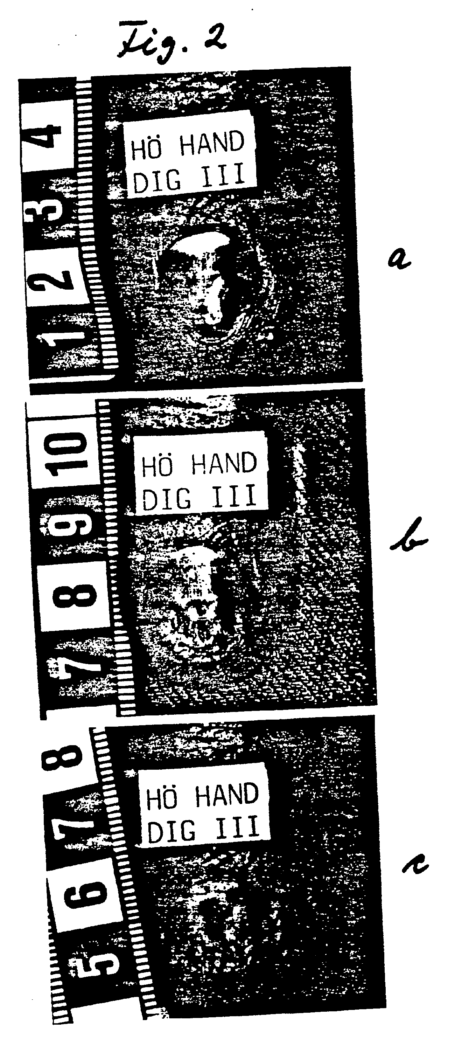

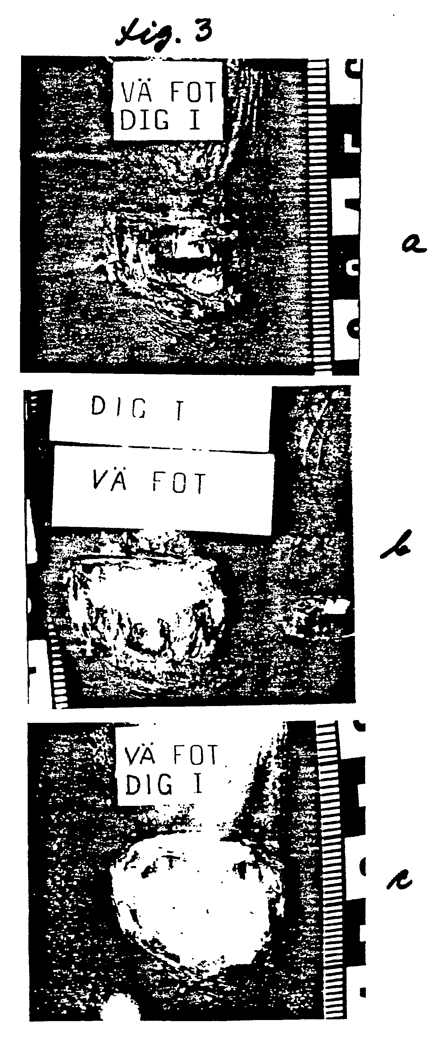

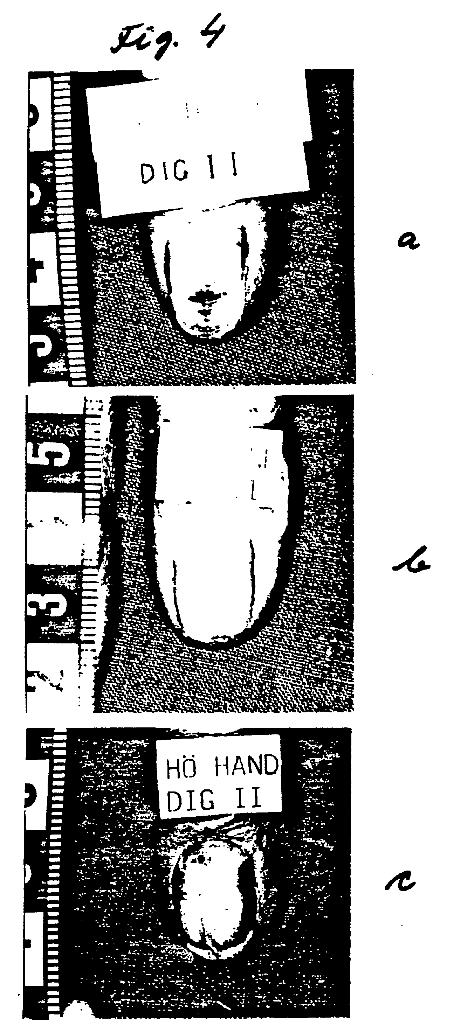

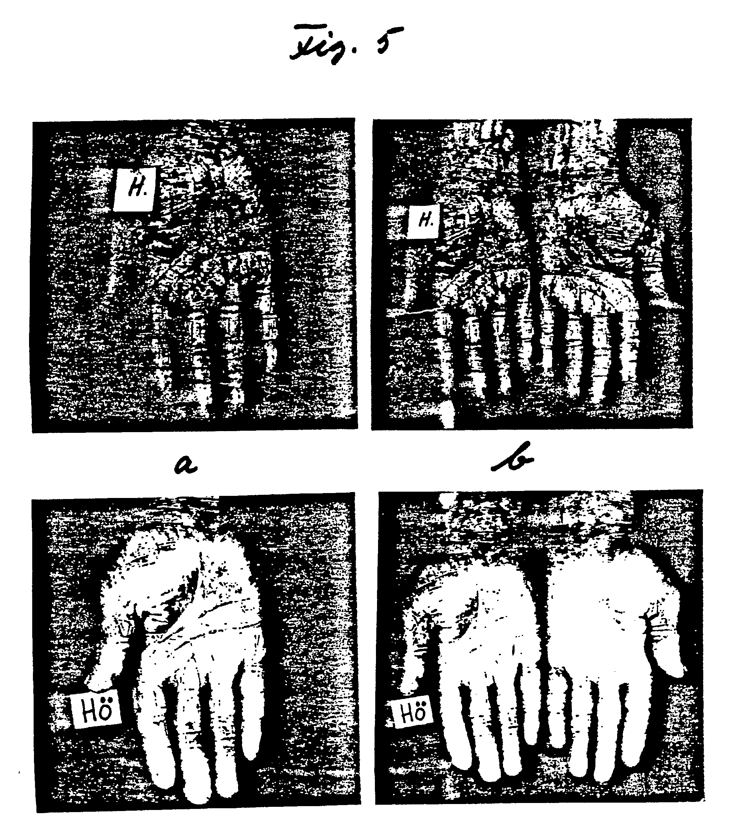

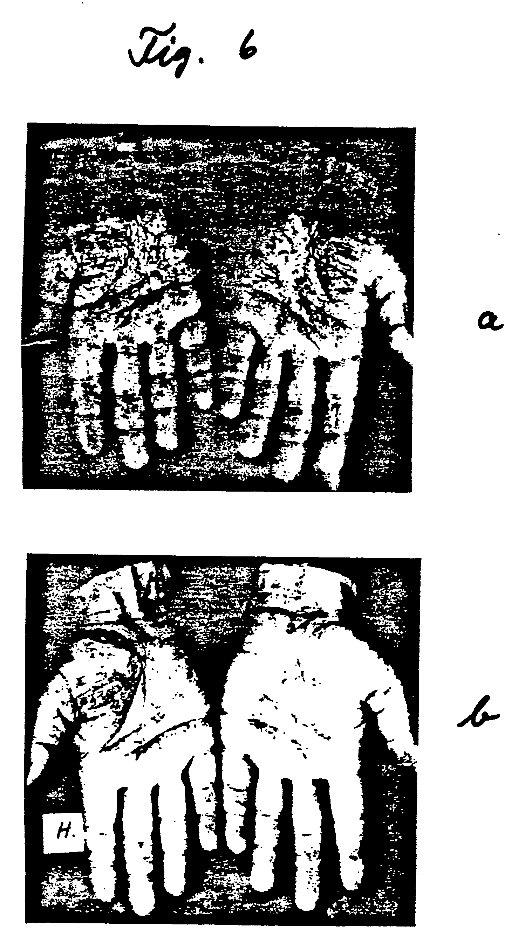

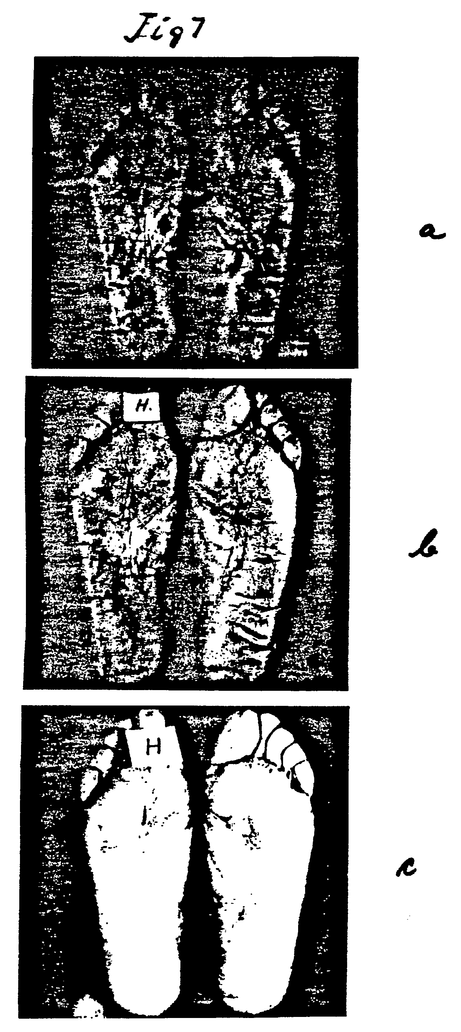

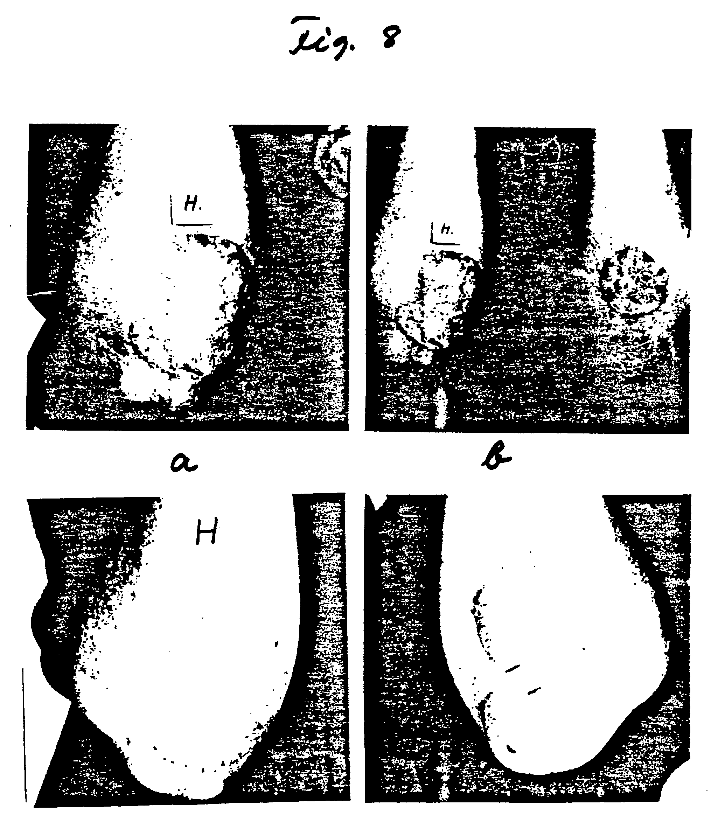

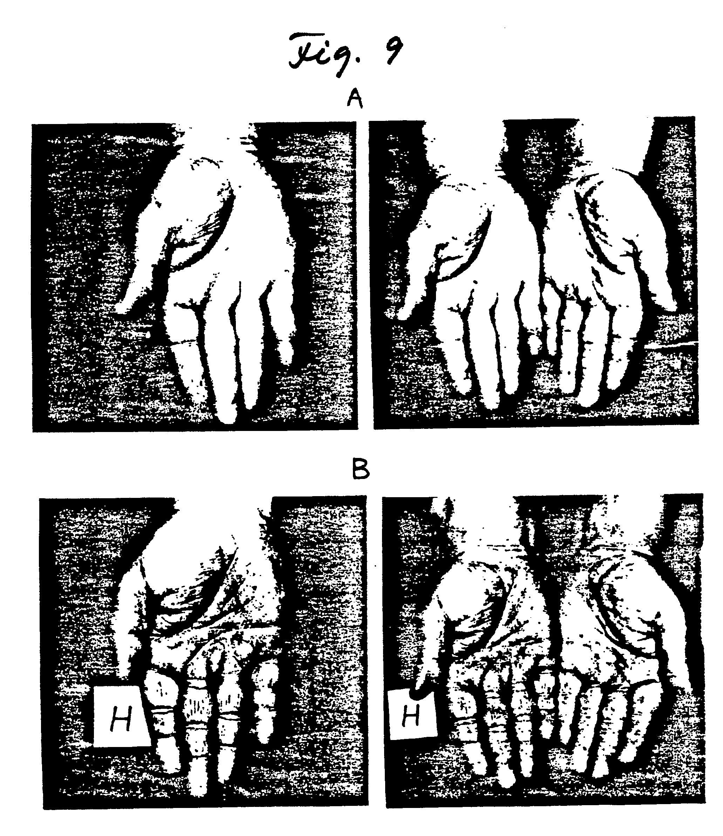

Fig. 1 Onychomycosis

Onychomycosis for 3 years. Treatment with imidazole solution for 1 year without effect.
a) Before treatment with the claimed preparation.
b) After 14 days of treatment.
c) After two months of treatment. The new nail has now started to develop proximally (at the top) and the discoloured part is simultaneously successively displaced downwards. This is evident if one compares pictures b and c.

Fig. 2 Onychomycosis

Onycho mycosis for about 3 years. Previous treatment with an imidazole solution made the nails somewhat softer but the patient did not notice any improvement.
a) Before treatment with the claimed preparation.
b) After 14 days of treatment.
c) After one month of treatment.

Fig. 3 Onychomycosis

a) Before treatment with the claimed preparation.
b) Example of the potent nail-disintegrating effect of the preparation after one month.
c) After two months. The infected nail substance is displaced and a new nail which cannot be reinfected starts to grow.

Fig. 4

Onychia About 3 years' damage of the nail plate. The impression has been newly formed as the nail has grown. Probably matrix damage in connection with hand eczema.
a) Before treatment with the claimed preparation.
b) Treatment results after 1.5 months.
c) Treatment results after 2.5 months.

Fig. 5

Toxic traumatic iterative hand eczema

Hand eczema since 1969.

Hand eczema before the treatment. The claimed preparation was applied to the right hand. (Marked with H).
A potent steroid cream (group III) Celestona valerate, was applied to the left hand. The hand eczema was somewhat more pronounced on the right hand from the beginning.
a) The result after 14 days of treatment with the claimed preparation. (Right hand (Hö) bottom picture).
b) The result after 14 days of treatment with a potent corticosteroid (group III), Celastona valerate. (Left hand bottom picture).

Fig. 6

Tylotic eczema

Chronic eczema on both palms. Heredity for psoriasis.
a) The right hand (marked with H) was treated with the claimed preparation. The left hand was treated with a potent corticosteroid in combination with salicylic acid (Diprosalic ointment).
b) The treatment results after one month. Somewhat better results in the right hand palm after treatment.

Fig. 7

Nickel eczema, psoriasis, hyperkeratosis, rhagades

a) A woman with chronic nickel eczema and psoriasis for several years. Thick hyperkeratosis with rhagades on both soles. Worse on the right side, more pain when walking. The right foot, marked A, was treated with the claimed preparation. The left foot was simultaneously treated with a potent steroid ointment (group III), Betnovate in combination with salicylic Essex cream.
b) The results after almost one month of treatment.
c) The result after slightly more than two months of treatment. The right side is remarkably improved. Only a fold of the skin now remains of the deep, painful chapped formation. The pain has disappeared from the right foot.

Fig. 8

Psoriasis

A patient with psoriasis since 1967. The right side (marked H) was treated with the claimed preparation. The left hand side (not marked) was treated with a potent steroid ointment (group III) in combination with salicylic acid (Diprosalic). In combination, further keratolytic treatment on the left side with salicylic Diachylon 5%.
a) The results after 14 days of treatment with the claimed preparation. The skin in the centre is healed.
b) The results after 14 days of treatment with Diprosalic and salicylic Diachylon.

Fig. 9

Tinea man. (fungus infection)

A chronic fungus infection on the palm of the right hand caused by Trichophyton rubrum.
A.
The results after one month of treatment.
B.
The palm of the right hand was healed at that time.

**Claims**

1. A composition active for the treatment of hyperkeratotic skin diseases, seborrheic eczema, dermatomycosis and onychomycosis, thickened and chapped skin, comprising propylene glycol and/or polyethylene glycol and urea as active main components, and optionally other active substances and additives, **characterized** in that the composition contains

40-80%    by weight of propylene glycol or propylene glycol and polyethylene glycol
5-20%     by weight of urea and
0-55%     by weight of other active substances and/or additives.

2. A composition according to claim 1, **characterized** in that it contains :

50-80%    by weight of propylene glycol or propylene glycol and polyethylene glycol
5-20%     by weight of urea and

11

0-45%     by weight of other active substances and/or additives.

3. A composition according to claim 1 or 2, **characterized** in that the further active substances and additives are

| 0-10% | by weight of glycerol |
|---|---|
| 0-5% | by weight of gel-forming agent |
| 0-20% | by weight of lactic acid |
| 0-55% | by weight of $C_{1-4}$-alcohols |
| 0-2% | by weight of glucocorticoids such as hydrocortisone or fluorinated corticosteroids |
| 0-2% | by weight of antimycotic substances such as imidazole derivatives |
| 0-10% | by weight of collodion |
| 0-10% | by weight of salicylic acid |
| 0-1% | by weight of vitamin A |
| 0-1% | by weight of vitamin A acid or derivatives thereof. |

4. A composition according to any of claims 1 and 3, **characterized** in that the further active substance is 10% by weight of lactic acid.

5. A composition according to any of claims 1 and 3, **characterized** in that the further additive is 30-40% by weight of ethanol.

6. A composition according to claim 1 to be used as penetration promoting solvent for glucocorticoids or antimycotic substances.

7. A composition according to claim 1, in the form of a solvent, gel, ointment or cream, preferably in the form of a solvent or a gel.

**Patentansprüche**

1. Zusammensetzung zur Behandlung von hyperkeratotischen Hautkrankheiten, seborrhoeischen Exzemen, von Dermatomycose und Onychomycose, von verdickter und rissiger Haut, umfassend Propylenglykol und/oder Polyethylenglykol und Harnstoff als wirksame Hauptkomponenten und gegebenenfalls andere Wirkverbindungen und Additive, dadurch **gekennzeichnet**, dass die Zusammensetzung

| 40 bis 80 Gew.% | Propylenglykol oder Propylenglykol und Polyethylenglykol, |
|---|---|
| 5 bis 20 Gew.% | Harnstoff und |
| 0 bis 55 Gew.% | an anderen Wirkverbindungen und/oder Additiven enthält. |

2. Zusammensetzung nach Anspruch 1, dadurch **gekennzeichnet**, dass sie

| 50 bis 80 Gew.% | Propylenglykol oder Propylenglykol und Polyethylenglykol |
|---|---|
| 5 bis 20 Gew.% | Harnstoff und |
| 0 bis 45 Gew.% | an anderen Wirkverbindungen und/oder Additiven enthält. |

3. Zusammensetzung nach Anspruch 1 oder 2, dadurch **gekennzeichnet**, dass die weiteren Wirkverbindungen und Additive

| 0 bis 10 Gew.% | Glyzerin |
|---|---|
| 0 bis 5 Gew.% | an gelbildendem Mittel |
| 0 bis 20 Gew.% | Milchsäure |
| 0 bis 55 Gew.% | $C_{1-4}$-Alkohole |
| 0 bis 2 Gew.% | Glucocorticoide, wie Hydrocortison, oder fluorierte Corticosteroide |
| 0 bis 2 Gew.% | antimykotische Verbindungen, wie Imidazolderivate |
| 0 bis 10 Gew.% | Kollodium |
| 0 bis 10 Gew.% | Salicylsäure |
| 0 bis 1 Gew.% | Vitamin A |
| 0 bis 1 Gew.% | Vitanim A-Säure oder Derivate hiervon sind. |

4. Zusammensetzung nach einem der Ansprüche 1 bis 3, dadurch **gekennzeichnet**, dass die weitere Wirkverbindung 10 Gew.% Milchsäure ist.

5. Zusammensetzung nach einem der Ansprüche 1 bis 3, dadurch **gekennzeichnet**, dass das weitere Additiv 30 bis 40 Gew.% Ethanol ist.

6. Zusammensetzung nach Anspruch 1 zur Verwendung als die Penetration förderndes Lösungsmittel für Glucocorticoide oder antimycotische Verbindungen.

7. Zusammensetzung nach Anspruch 1 in Form eines Lösungsmittels, eines Gels, einer Salbe oder Creme, vorzugsweise in Form eines Lösungsmittels oder eines Gels.


## Revendications

1. Composition active destinée au traitement d'affections dermatologiques de type hyperkératose, eczéma séborrhéique, dermatomycose et onychomycose, de l'épaississement et de gerçures de l'épiderme, comprenant du propylène-glycol et/ou du polyéthylène-glycol et de l'urée comme principaux constituants, et facultativement d'autres substances actives et additifs,
caractérisée en ce que la composition contient

40-80% en poids de propylène-glycol ou de propylène-glycol et de polyéthylène-glycol
5-20% en poids d'urée et
0-55% en poids d'autres substances actives et/ou additifs.

2. Composition selon la revendication 1, caractérisée en ce qu'elle contient :

50-60% en poids de prolylène-glycol ou de propylène-glycol et de polyéthylène-glycol
5-20% en poids d'urée et
0-45% en poids d'autres substances actives et/ou additifs.

3. Composition selon la revendication 1 ou 2, caractérisée en ce que les autres substances actives et additifs sont

0-10% en poids de glycérol
0-5% en poids d'agent formant un gel
0-20% en poids d'acide lactique
0-55% en poids d'alcools $C_{1-4}$
0-2% en poids de glucocorticoïdes tels que de l'hydrocortisone ou des corticostéroïdes fluorés
0-2% en poids de substances antimycotiques telles que les dérivés de l'imidazole.
0-10% en poids de collodion
0-10% en poids d'acide salicylique
0-1% en poids de vitamine A
0-1% en poids d'acide de vitamine A ou de ses dérivés.

4. Composition selon l'une quelconque des revendications 1 et 3, caractérisée en ce que l'autre substance active est constituée par 10% en poids d'acide lactique.

5. Composition selon l'une quelconque des revendications 1 et 3, caractérisée en ce que l'autre additif est constitué par 30-40% en poids d'éthanol.

6. Composition selon la revendication 1 à utiliser comme solvant facilitant la pénétration pour les glucocorticoïdes ou les substances antimycotiques.

7. Composition selon la revendication 1 sous forme d'un solvant d'un gel d'une pommade ou d'une crème, de préférence sous la forme d'un solvant ou d'un gel.

Fig. 1

a

b

c

Fig. 2

*Fig.* 3

*Fig. 4*

a

b

c

Fig. 5

Fig. 6

a

b

Fig 7

Fig. 8

Fig. 9

A

B